# EUROPEAN PATENT APPLICATION

(11) **EP 4 722 735 A1**
(43) Date of publication of application: **08.04.2026**
(21) Application number: 23939724.3
(22) Date of filing: 02.06.2023
(51) Int. Cl.: G01P 15/00

(54) **WALKING MOTION VISUALIZATION DEVICE AND WALKING MOTION VISUALIZATION METHOD, AND ALIGNED STATE DETERMINATION DEVICE AND ALIGNED STATE DETERMINATION METHOD**

(71) Applicant: Kao Corporation, Tokyo 103-8210 (JP)
(72) Inventor: UEDA, Tomoya, Tokyo 103-8210 (JP); SUDO, Motoki, Tokyo 103-8210 (JP); YAMASHIRO, Yukari, Tokyo 103-8210 (JP)
(74) Representative: Hoffmann Eitle
(86) International application number: PCT/JP2023/020623
(87) International publication number: WO 2024/247252

(57) **Abstract**

A walking motion visualization apparatus includes an acceleration data acquire for acquiring, from an acceleration sensor disposed on a median line that is a center line between left and right sides of a body of a subject, stationary state acceleration data for determining a direction of gravity in a state where the subject is stationary and determination acceleration data that is acceleration data when the subject is walking in order to determine whether the acceleration sensor is in a directly opposing state with respect to the median line or not, a determiner for determining whether the acceleration sensor is in a directly opposing state with respect to the median line or not, based on the acquired stationary state acceleration data and determination acceleration data, a walking acceleration data acquire for acquiring walking acceleration data from the acceleration sensor while the subject is walking when the determiner determines that the acceleration sensor is in a directly opposing state with respect to the median line, a walking motion analyzer for analyzing a walking motion of the subject, based on the acquired walking acceleration data, and a display controller for displaying a walking motion model image of the subject, based on an analysis result of the walking motion analyzer.

## Description

### Technical Field

The present invention relates to a walking motion visualization apparatus and a walking motion visualization method, and a directly opposing state determination apparatus and a directly opposing state determination method.

### Background Art

In the above technical field, Patent Document 1 discloses a technique for quantitatively calculating a physical quantity corresponding to a position of a waist of a measurement subject during walking by using an acceleration sensor attached on a median line of the waist of the measurement subject (claim 1 and the like).

### Citation List

### Patent Literature

Patent Document 1: US2016/038059A1

### Summary of Invention

In order to solve the above problem, a walking motion visualization apparatus according to the present invention includes
an acceleration data acquire for acquiring, from an acceleration sensor disposed on a median line that is a center line between left and right sides of a body of a subject,
   stationary state acceleration data for determining a direction of gravity in a state where the subject is stationary and
   determination acceleration data that is acceleration data when the subject is walking in order to determine whether the acceleration sensor is in a directly opposing state with respect to the median line or not,
a determiner for determining whether the acceleration sensor is in a directly opposing state with respect to the median line or not, based on the acquired stationary state acceleration data and determination acceleration data,
a walking acceleration data acquire for acquiring walking acceleration data from the acceleration sensor while the subject is walking when the determiner determines that the acceleration sensor is in a directly opposing state with respect to the median line,
a walking motion analyzer for analyzing a walking motion of the subject, based on the acquired walking acceleration data, and
a display controller for displaying a walking motion model image of the subject, based on an analysis result of the walking motion analyzer.

In order to solve the above problem, a walking motion visualization method according to the present invention includes,
an acceleration data acquisition step of acquiring, from an acceleration sensor disposed on a median line that is a center line between left and right sides of a body of a subject,
   stationary state acceleration data for determining a direction of gravity in a state where the subject is stationary and
   determination acceleration data that is acceleration data when the subject is walking in order to determine whether the acceleration sensor is in a directly opposing state with respect to the median line or not,
a determination step of determining whether the acceleration sensor is in a directly opposing state with respect to the median line or not, based on the acquired stationary state acceleration data and determination acceleration data,
a walking acceleration data acquisition step of acquiring walking acceleration data from the acceleration sensor while the subject is walking when it is determined in the determination step that the acceleration sensor is in a directly opposing state with respect to the median line,
a walking motion analysis step of analyzing a walking motion of the subject, based on the acquired walking acceleration data, and
a display control step of displaying a walking motion model image of the subject, based on an analysis result in the walking motion analysis step.

In order to solve the above problem, a directly opposing state determination apparatus according to the present invention includes,
an acceleration data acquire for acquiring, from an acceleration sensor disposed on a median line that is a center line between left and right sides of a body of a subject,
   stationary state acceleration data for determining a direction of gravity in a state where the subject is stationary and
   determination acceleration data that is acceleration data when the subject is walking in order to determine whether the acceleration sensor is in a directly opposing state with respect to the median line or not, and
a determiner for determining whether the acceleration sensor is in a directly opposing state with respect to the median line or not, based on the acquired stationary state acceleration data and determination acceleration data.

In order to solve the above problem, a directly opposing state determination method according to the present invention includes,
an acceleration data acquisition step of acquiring, from an acceleration sensor disposed on a median line that is a center line between left and right sides of a body of a subject,
   stationary state acceleration data for determining a direction of gravity in a state where the subject is stationary and
   determination acceleration data that is acceleration data when the subject is walking in order to determine whether the acceleration sensor is in a directly opposing state with respect to the median line or not, and
a determination step of determining whether the acceleration sensor is in a directly opposing state with respect to the median line or not, based on the acquired stationary state acceleration data and determination acceleration data.

### Brief Description of Drawings

[Fig. 1A] Fig. 1A is a diagram showing an outline of analysis of a walking motion of a walking motion visualization apparatus according to a first embodiment of the present invention.
[Fig. 1B] Fig. 1B is a diagram showing a display example (horizontal bar graph) of analysis results of walking motions of the walking motion visualization apparatus according to the first embodiment of the present invention.
[Fig. 1C] Fig. 1C is a diagram showing a display example (pie chart) of analysis results of walking motions of the walking motion visualization apparatus according to the first embodiment of the present invention.
[Fig. 1D] Fig. 1D is a diagram showing a display example (pendulum graph) of analysis results of walking motions of the walking motion visualization apparatus according to the first embodiment of the present invention.
[Fig. 1E] Fig. 1E is a diagram showing a display example (balance graph) of analysis results of walking motions of the walking motion visualization apparatus according to the first embodiment of the present invention.
[Fig. 1F] Fig. 1F is a diagram showing a display example (radar chart) of analysis results of walking motions of the walking motion visualization apparatus according to the first embodiment of the present invention.
[Fig. 1G] Fig. 1G is a diagram showing an example of a case where an analysis result of a walking motion of the walking motion visualization apparatus according to the first embodiment of the present invention is displayed by a skeleton model image.
[Fig. 1H] Fig. 1H is a diagram showing another example of a case where an analysis result of a walking motion of the walking motion visualization apparatus according to the first embodiment of the present invention is displayed by a skeleton model image.
[Fig. 1I] Fig. 1I is a diagram showing still another example of a case where an analysis result of a walking motion of the walking motion visualization apparatus according to the first embodiment of the present invention is displayed by a skeleton model image.
[Fig. 2] Fig. 2 is a block diagram showing the configuration of the walking motion visualization apparatus according to the first embodiment of the present invention.
[Fig. 3] Fig. 3 is a diagram showing an example of a joint angle table included in the walking motion visualization apparatus according to the first embodiment of the present invention.
[Fig. 4] Fig. 4 is a diagram showing a hardware configuration of the walking motion visualization apparatus according to the first embodiment of the present invention.
[Fig. 5A] Fig. 5A is a flowchart showing a processing procedure of the walking motion visualization apparatus according to the first embodiment of the present invention.
[Fig. 5B] Fig. 5B is a flowchart showing a directly opposing state determination process of the walking motion visualization apparatus according to the first embodiment of the present invention.
[Fig. 5C] Fig. 5C is a flowchart showing a walking motion analysis process of the walking motion visualization apparatus according to the first embodiment of the present invention.
[Fig. 6] Fig. 6 is a block diagram showing the configuration of a walking motion visualization apparatus according to a second embodiment of the present invention.

### Description of Embodiments

In the technique disclosed in Patent Document 1, a predetermined physical quantity is calculated from a measurement value of the acceleration sensor in a state where the acceleration sensor is attached on the median line of the waist of the measurement subject, and it is not determined whether the acceleration sensor is in a state where the acceleration sensor can output a correct measurement value or not. For this reason, the evaluation of a walking posture of the measurement subject performed by the physical quantity calculated using the measurement value output from the acceleration sensor has low reliability.

Hereinafter, embodiments of the present invention will be described with reference to the drawings. However, configurations, numerical values, processing flows, functional elements, and the like described in the following embodiments are merely examples and can be freely modified or changed, and the technical scope of the present invention is not intended to be limited to the following description.

### First Embodiment

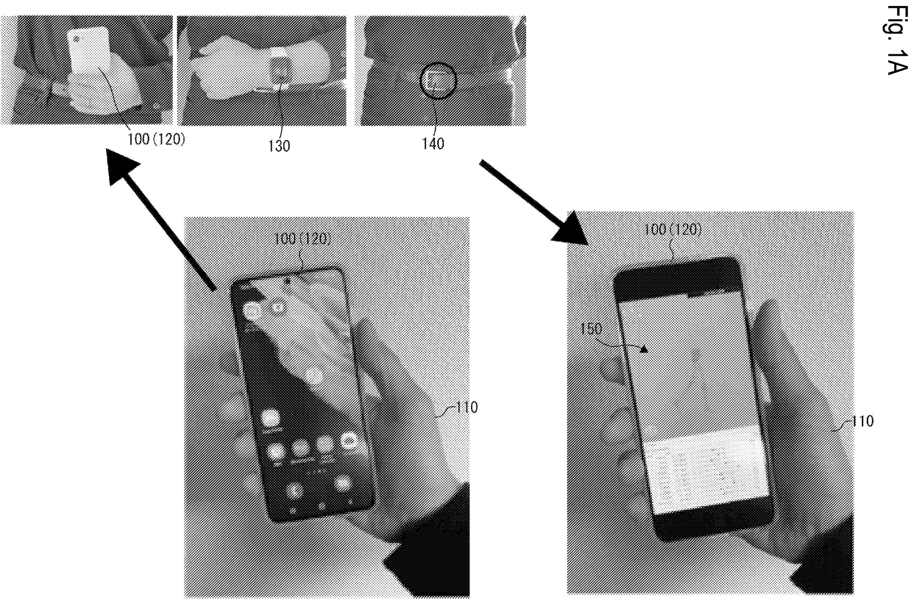

A walking motion visualization apparatus 100 according to a first embodiment of the present invention will be described with reference to Fig. 1A to Fig. 5C. The walking motion visualization apparatus 100 is an apparatus for visualizing a walking motion, a walking speed, and the like of a subject 110 by an animation image or the like using acceleration data obtained from an acceleration sensor built into a portable terminal or the like. Note that, in the following description, a portable terminal such as a smartphone 120 is described as an example of the walking motion visualization apparatus 100, but some or all of the functions of the walking motion visualization apparatus 100 may be implemented by a general-purpose computer apparatus such as a server.

The walking motion such as a walking speed not only reflects general health conditions and functional ability, but also serves as various predictive factors such as frailty and decline in Activities of Daily Living (ADL). For this reason, a walking motion (walking speed, and the like) is also referred to as a sixth vital sign, as well as the blood pressure, the body temperature, and the like. For example, in order to keep a walking speed and to continue to walk with one's own feet, it is important to walk with a walking motion using the body correctly without strain on the body.

For example, in order to walk with a large stride, it is important to move main joints such as hip joints, knee joints, and ankle joints normally, as well as to move muscles of the whole body. That is, when the way of walking (walking motion) is known, a part that is not correctly used in a muscle, a joint, or the like is known, or a part that is excessively used is known, and thus this can lead to not only training and exercise guidance but also prevention of injury.

Here, examples of a method of measuring the way of walking (walking motion) include a motion analysis system that represents the walking posture of the whole body, a depth sensor-camera (Kinect), a wearable suit, a method of attaching a plurality of sensors to the body (motion capture or the like), and the like. These methods are characterized in that a joint angle of the body can be measured, but they require special measurement equipment and are limited to measurement situations, and thus these methods are methods for measuring the way of walking in unusual situations.

In order to measure a walking motion, a walking posture, and the like in daily life, it is necessary to classify the motion, posture, and the like of the body such as the left-right balance of the body and the forward inclination of the body by the acceleration sensor attached to the waist or the like of the subject 110. However, whether the acceleration sensor is correctly attached to the waist or the like or not is left to the determination of the subject himself or herself. For this reason, in order to correctly measure a walking motion and the like of the subject 110, first, it is necessary to attach the acceleration sensor to the body of the subject 110 at a position suitable for measuring a walking motion, a walking posture, and the like.

Most of output results obtained by the above-described measurement methods show the degree of lateral swing of the body, the degree of forward inclination, the degree of backward inclination, and the like, and show walking motions and the like using predetermined patterns similar to some walking models. For this reason, the measurement method has not been able to specify the cause of the current walking motion or the like.

Thus, in the walking motion visualization apparatus 100 according to the present embodiment, first, it is determined whether the acceleration sensor is attached on the median line or not, which is the center of the body of the subject 110 in the left-right direction. The walking motion visualization apparatus 100 measures acceleration data of the subject 110 during walking in a state where the acceleration sensor is attached on the median line. Next, the walking motion visualization apparatus 100 selects acceleration data approximate to the measured acceleration data from a predetermined database, estimates a joint angle of the subject 110 during walking, and images the walking motion of the subject 110. When the acceleration sensor is on the median line, the walking motion visualization apparatus 100 can notify the subject 110 of a walking start instruction to prompt the subject to start measurement.

Note that, as shown in Fig. 1A, in the present embodiment, an acceleration sensor built into the smartphone 120, a smartwatch 130, or the like, or an acceleration sensor included in an activity meter 140 can be used. The activity meter 140 can be used by being built into a belt or the like, for example. In addition, the acceleration sensor may be a sensor that is not built into the smartphone 120 or the smartwatch 130 but is independently provided. In this case, the acceleration sensor, the smartphone 120, and the like exchange measurement data by wireless communication or wired communication.

In the measurement of the walking motion in the walking motion visualization apparatus 100, first, the walking motion visualization apparatus 100 instructs the subject 110 by a sound, an image, or the like so that the subject 110 holds the smartphone 120 or the like on the median line of the body of the subject 110. When the time during which the data from the acceleration sensor is a constant value continues for approximately two seconds or three seconds, the walking motion visualization apparatus 100 determines that the subject 110 has held the smartphone 120 or the like on the median line, and in this state, the walking motion visualization apparatus 100 acquires acceleration data and determines the direction of gravity. Note that when the subject 110 holds the smartphone 120, the smartphone 120 may be brought into contact with the abdomen of the subject 110. Further, when the subject 110 uses the smart watch 130, the subject 110 may bring one of the left and right wrists to which the smart watch 130 is attached to the position of the abdomen of the subject 110. In addition, when the subject 110 uses the activity meter 140, a belt to which the activity meter 140 is attached may be wound around the abdomen of the subject 110. Note that any method other than the method described here may be adopted as long as the subject 110 can hold or dispose the acceleration sensor so as to be in the directly opposing state on the median line.

When the smartphone 120 or the like is in a directly opposing state, the walking motion visualization apparatus 100 instructs the subject 110 to start walking by, for example, vibration or sound, acquires acceleration data of the subject 110 during walking, and analyzes the walking motion of the subject 110 using the acceleration data of the subject 110 during walking. When the analysis of the walking motion of the subject 110 ends, the walking motion visualization apparatus 100 displays the walking motion of the subject 110 as a walking motion model image 150 on, for example, a display of the smartphone 120. Note that the walking motion visualization apparatus 100 may be for instructing the subject 110 to end the walking by a sound when it is detected that a sufficient amount of acceleration data necessary for analysis has been obtained.

The subject 110 can recognize his or her current walking motion by viewing the walking motion model image 150. The walking motion visualization apparatus 100 can also display, to the subject 110, a defect, an improvement, an improvement method, advice, and the like of the walking motion of the subject 110 together with the walking motion model image 150.

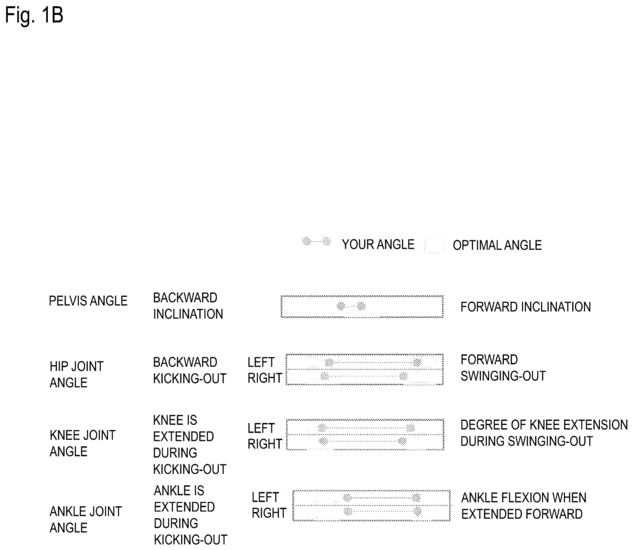

Then, the subject 110 practices the walking motion according to the displayed improvement method or the like, thereby enabling the subject 110 to naturally learn the walking motion suitable for the subject 110 himself or herself. The walking motion model image 150, and the improvement method and advice, which are displayed together with the walking motion model image 150, can be displayed by various methods, for example, as shown in Fig. 1B to Fig. 1I.

For example, as shown in Fig. 1B, the state (angle) of a pelvis angle, a hip joint angle, a hip joint angle, an ankle joint angle, and the like can be displayed using a horizontal bar graph. Further, these standard values can be displayed together in the horizontal bar graph.

The walking motion visualization apparatus 100 can display, for example, the pelvis angle, that is, the state (angle) of the posture of the subject 110 between the forward inclination and the backward inclination. The walking motion visualization apparatus 100 can also display the pelvis angle of the subject 110 together with an optimal angle range indicating the range of values within which the pelvis angle of the subject 110 should fall.

Similarly, regarding the hip joint angle, the walking motion visualization apparatus 100 can display the state (angle) of the posture of the subject 110. Regarding the hip joint angle, the walking motion visualization apparatus 100 can display the degree of forward swing and the degree of backward swing of left and right hip joint angles. Regarding the left and right hip joint angles, the walking motion visualization apparatus 100 can also display an optimal angle range indicating a range within which the hip joint angle of the subject 110 should fall.

Furthermore, regarding a knee joint angle, the walking motion visualization apparatus 100 can also display the state (angle) of the posture of the subject 110. Regarding the knee joint angle, the walking motion visualization apparatus 100 can display, for the left and right knee joint angles, the degree of knee extension at the time of swinging-out, the degree of knee extension at the time of kicking-out, and the like. Regarding the left and right knee joint angles, the walking motion visualization apparatus 100 can also display an optimal angle range indicating a range within which the knee joint angle of the subject 110 should fall.

Regarding the ankle joint angle, the walking motion visualization apparatus 100 can also display the state (angle) of the posture of the subject 110. Regarding the ankle joint angle, the walking motion visualization apparatus 100 displays, for the left and right ankle joint angles, the degree of ankle flexion when the foot is extended forward and whether the ankle is extended during push-off. Regarding the left and right ankle joint angles, the walking motion visualization apparatus 100 can also display an optimal angle range indicating a range within which the ankle joint angles of the subject 110 should fall.

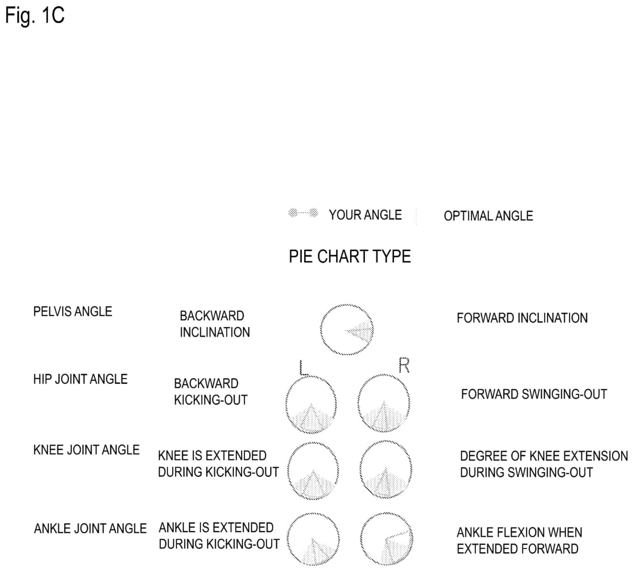

Next, as shown in Fig. 1C, the walking motion visualization apparatus 100 can display the states (angles) of a pelvis angle, a hip joint angle, a knee joint angle, an ankle joint angle, and the like using pie charts. Further, these standard values can be displayed together on the pie charts.

The walking motion visualization apparatus 100 can display, for example, the degree of forward inclination or backward inclination of the pelvis angle using one pie chart. By displaying the pie chart, the subject 110 can visually recognize at a glance the state of the pelvis angle of the subject himself or herself. Note that, as will be described below, the subject 110 can visually recognize other joints.

Similarly, regarding the hip joint angle, the walking motion visualization apparatus 100 can display the state (angle) of the posture of the subject 110 using two circular flags. Regarding the hip joint angles, the walking motion visualization apparatus 100 displays, for the left and right hip joint angles, the degree of forward swing and the degree of backward swing using two circular flags. Regarding the left and right hip joint angles, the walking motion visualization apparatus 100 can also display, in the same pie chart, an optimal angle range indicating a range within which the hip joint angle of the subject 110 should fall.

Furthermore, regarding the knee joint angle, the walking motion visualization apparatus 100 can display the state (angle) of the posture of the subject 110 using two pie charts. Regarding the knee joint angle, the walking motion visualization apparatus 100 displays, for the left and right knee joint angles, the degree of knee extension at the time of swinging-out, the degree of knee extension at the time of kicking-out, and the like. Regarding the left and right knee joint angles, the walking motion visualization apparatus 100 can also display an optimal angle range indicating a range within which the knee joint angle of the subject 110 should fall.

In addition, regarding the ankle joint angle, the walking motion visualization apparatus 100 can display the state (angle) of the posture of the subject 110 using two pie charts. Regarding the ankle joint angle, the walking motion visualization apparatus 100 displays, for the left and right ankle joint angles, the degree of ankle flexion when the foot is extended forward and whether the ankle is extended during push-off. Regarding the left and right ankle joint angles, the walking motion visualization apparatus 100 can display an optimal angle range indicating a range within which the ankle joint angles of the subject 110 should fall.

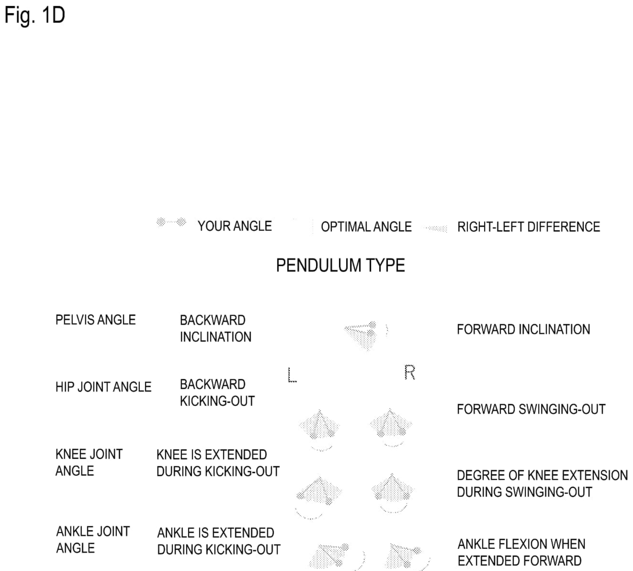

Next, as shown in Fig. 1D, the walking motion visualization apparatus 100 can display the states (angles) of a pelvis angle, a hip joint angle, a knee joint angle, an ankle joint angle, and the like using pendulum fluff, and can also display standard values, left-right differences, and the like of these angles. The walking motion visualization apparatus 100 can display, for example, the degree of forward inclination or backward inclination of the pelvis angle using one pendulum graph. By displaying the pendulum graph, the subject 110 can visually recognize at a glance the state of the pelvis angle of the subject himself or herself. Note that, as will be described below, the subject 110 can visually recognize other joints.

Similarly, regarding the hip joint angle, the walking motion visualization apparatus 100 displays the state (angle) of the posture of the subject 110, that is, the degree of forward swing, the degree of backward swing, and the like, using two pendulum graphs. Regarding the left and right hip joint angles, the walking motion visualization apparatus 100 can also display, in the same pendulum graph, an optimal angle range indicating a range within which the hip joint angle of the subject 110 should fall. In addition, when there is a difference between the left and right hip joint angles, the walking motion visualization apparatus 100 can also display the difference.

Furthermore, regarding the knee joint angle, the walking motion visualization apparatus 100 can display the state (angle) of the posture of the subject 110 using two pendulum graphs. Regarding the knee joint angle, the walking motion visualization apparatus 100 displays, for the left and right knee joint angles, the degree of knee extension at the time of swinging-out, the degree of knee extension at the time of kicking-out, and the like. Regarding the left and right knee joint angles, the walking motion visualization apparatus 100 can display an optimal angle range indicating a range within which the knee joint angle of the subject 110 should fall.

In addition, regarding the ankle joint angle, the walking motion visualization apparatus 100 can display the state (angle) of the posture of the subject 110 using two pendulum graphs. Regarding the ankle joint angle, the walking motion visualization apparatus 100 displays, for the left and right ankle joint angles, the degree of ankle flexion when the foot is extended forward and whether the ankle is extended during push-off. Regarding the left and right ankle joint angles, the walking motion visualization apparatus 100 can display an optimal angle range indicating a range within which the ankle joint angles of the subject 110 should fall.

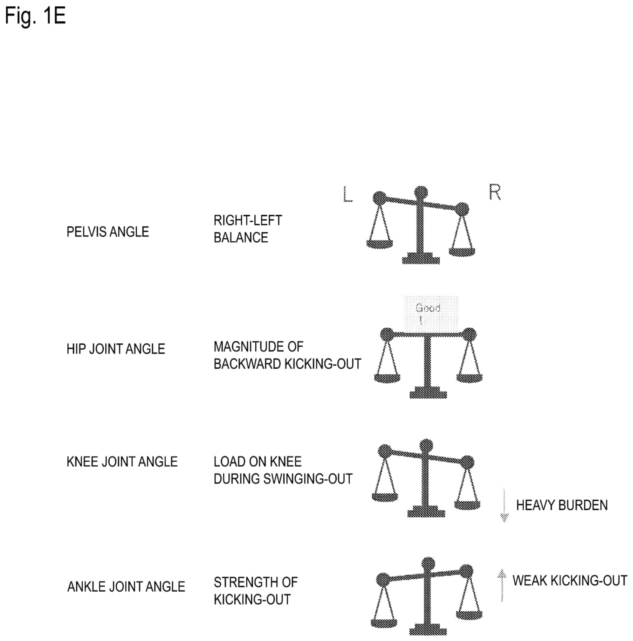

Next, as shown in Fig. 1E, the walking motion visualization apparatus 100 can display the states (angles) of a pelvis angle, a hip joint angle, a knee joint angle, an ankle joint angle, and the like using a balance graph. When the balance graph is used, the walking motion visualization apparatus 100 can display the subject 110 with emphasis on a difference between the left and right sides, the balance between the left and right sides, and the like.

The walking motion visualization apparatus 100 can display, for example, the front-back balance of the pelvis angle, that is, the degree of forward inclination and the degree of backward inclination using one balance graph. By displaying the balance graph, the subject 110 can visually recognize at a glance the state of the pelvis angle of the subject himself or herself. Note that, as will be described below, the subject 110 can visually recognize other joints.

Similarly, regarding the hip joint angle, the walking motion visualization apparatus 100 displays the state (angle) of the posture of the subject 110, that is, the degree of forward swing, the degree of backward swing, and the like, using one balance graph. For example, the walking motion visualization apparatus 100 can display a diagram of a well-balanced graph when there is no difference between the left and right knee joint angles of the subject 110 during walking, and can display the state of the subject 110 by various methods such as displaying the word "Good!".

Furthermore, regarding the knee joint angle, the walking motion visualization apparatus 100 can display the state (angle) of the posture of the subject 110 using one balance graph. Regarding the knee joint angle, the walking motion visualization apparatus 100 displays, for the left and right knee joint angles, the degree of knee extension at the time of swinging-out, the degree of knee extension at the time of kicking-out, and the like. Then, regarding the knee joint angle, for example, when the degree of knee extension at the time of swinging-out is larger as a result of comparing the degree of knee extension at the time of swinging-out and the degree of knee extension at the time of kicking-out, the walking motion visualization apparatus 100 displays a diagram in which the tray of the balance on the side of the degree of knee extension at the time of swinging-out is lowered in the balance graph. With such a display, the subject 110 can easily and reliably recognize that the burden on the degree of knee extension at the time of swinging-out is heavy.

In addition, regarding the ankle joint angle, the walking motion visualization apparatus 100 can display the state (angle) of the posture of the subject 110 using one balance graph. Regarding the ankle joint angle, the walking motion visualization apparatus 100 displays, for the left and right ankle joint angles, the degree of ankle flexion when the foot is extended forward and whether the ankle is extended during push-off. For example, the walking motion visualization apparatus 100 displays a diagram of a well-balanced balance when there is no difference between the front and back ankle joint angles of the subject 110 during walking. Furthermore, when the degree of extension of the ankle during kicking-out is larger than the degree of ankle flexion when the foot is extended forward, the walking motion visualization apparatus 100 displays a diagram in which the tray of the balance on the extension side of the ankle during kicking-out is lowered. In this manner, the subject 110 can easily and reliably recognize that kicking-out is strong at the time of kicking-out.

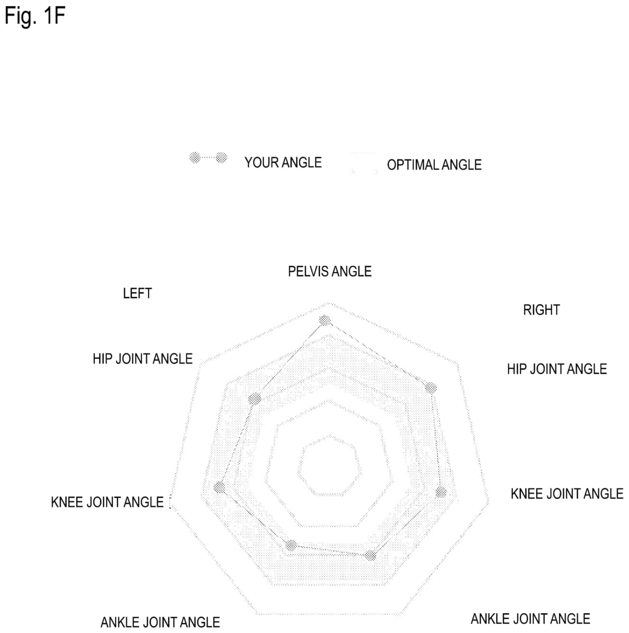

Next, as shown in Fig. 1F, the walking motion visualization apparatus 100 can display the states (angles) of a pelvis angle, a hip joint angle, a knee joint angle, an ankle joint angle, and the like using a radar chart. When the walking motion visualization apparatus 100 displays the states using the radar chart, for example, the walking motion visualization apparatus 100 displays the angle of a joint on the right side of the body of the subject 110 on the right side of the radar chart, and displays the angle of a joint on the left side of the body of the subject 110 on the left side of the radar chart. By displaying the angles in this manner, the subject 110 can recognize his or her own left-right difference and left-right balance at a glance.

The walking motion visualization apparatus 100 can display, for example, the degree of forward inclination and the degree of backward inclination of the pelvis angle on the radar chart. The walking motion visualization apparatus 100 also displays an optimal angle range indicating a range within which the posture of the subject 110 should fall.

Similarly, regarding the hip joint angle, the walking motion visualization apparatus 100 can display the state (angle) of the posture of the subject 110. Regarding the hip joint angle, the walking motion visualization apparatus 100 displays the degree of forward swing and the degree of backward swing of left and right hip joint angles. Regarding the left and right hip joint angles, the walking motion visualization apparatus 100 can display an optimal angle range indicating a range within which the hip joint angle of the subject 110 should fall.

Furthermore, regarding the knee joint angle, the walking motion visualization apparatus 100 can display the state (angle) of the posture of the subject 110. Regarding the knee joint angle, the walking motion visualization apparatus 100 can display, for the left and right knee joint angles, the degree of knee extension at the time of swinging-out, the degree of knee extension at the time of kicking-out, and the like. In the walking motion visualization apparatus 100, the state of the knee joint angle is displayed using the radar chart, and thus the subject 110 can recognize a difference and balance between the left and right knee joint angles at a glance. Note that the joint angle includes, in addition to the anteroposterior flexion and extension, lateral rotation and adduction and abduction. Further, when data is acquired for a neck joint, a shoulder joint, an elbow joint, and a wrist joint by using measurement data other than acceleration data in the median line, for example, data from an acceleration sensor mounted on the body of the subject, such as data from an acceleration sensor built into an earphone or a headphone, or data from an acceleration sensor of a wristwatch type biometric apparatus provided on the wrist, the operation state of the joint can be similarly displayed.

Regarding the ankle joint angle, the walking motion visualization apparatus 100 can also display the state (angle) of the posture of the subject 110. Regarding the ankle joint angle, the walking motion visualization apparatus 100 displays, for the left and right ankle joint angles, the degree of ankle flexion when the foot is extended forward and whether the ankle is extended during push-off. Regarding the left and right ankle joint angles, the walking motion visualization apparatus 100 can display an optimal angle range indicating a range within which the ankle joint angles of the subject 110 should fall. In this manner, in the walking motion visualization apparatus 100, the states of the respective joints are displayed using the radar chart, and thus the subject 110 can visually recognize defects and improvements of his or her walking motion, and can be motivated to improve his or her walking motion.

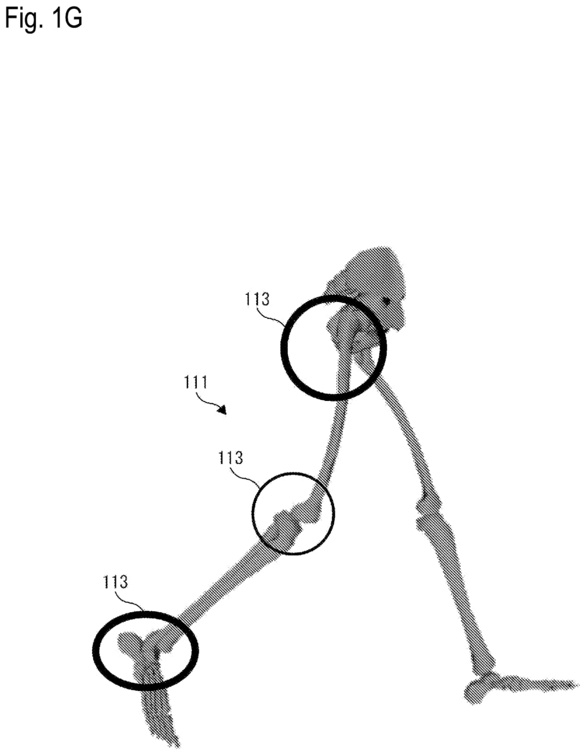

Next, a method of displaying an analysis result of a walking motion of the subject 110 will be described with reference to Fig. 1G to Fig. 1I. For example, as shown in Fig. 1G, the walking motion visualization apparatus 100 displays, on a skeleton model image 111 of the lower body of the subject 110, circles 113 at the positions of the joints that the subject 110 is caused to consciously move in order to improve a walking motion. The walking motion visualization apparatus 100 can further display information together with advice such as "a place to move more". Furthermore, for example, regarding the angle joint, when the joint of the right foot is less usable than the joint of the left foot, the walking motion visualization apparatus 100 can also display advice such as "kicking-out is required more because the right foot is less usable than the left foot".

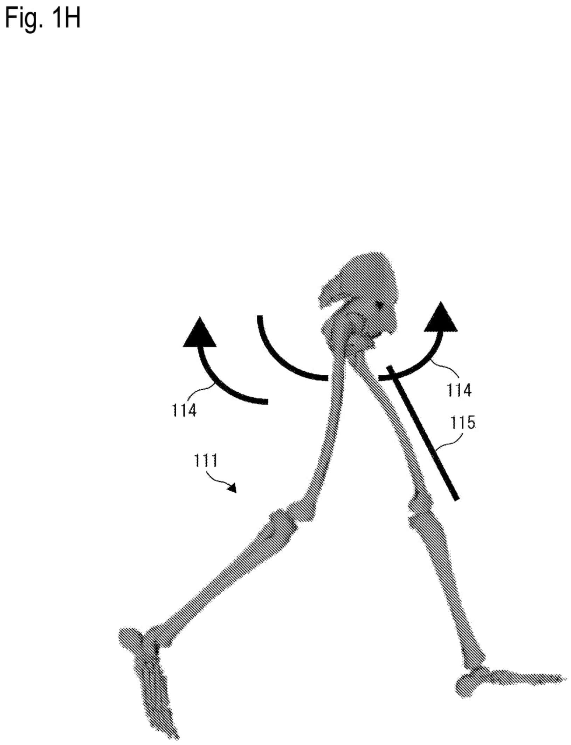

Further, for example, as shown in Fig. 1H, the walking motion visualization apparatus 100 displays an arrow 114 and a straight line 115 on the skeleton model image of the lower body of the subject 110. That is, the walking motion visualization apparatus 100 uses the arrow 114 to indicate the direction in which a joint, a muscle, or the like is to be moved and, for example, displays the straight line 115 next to the thigh to indicate the muscle under a load (the part to be trained). In this manner, the walking motion visualization apparatus 100 displays advice that helps improve a walking motion together, thereby allowing the subject 110 to improve the walking motion in accordance with the displayed advice.

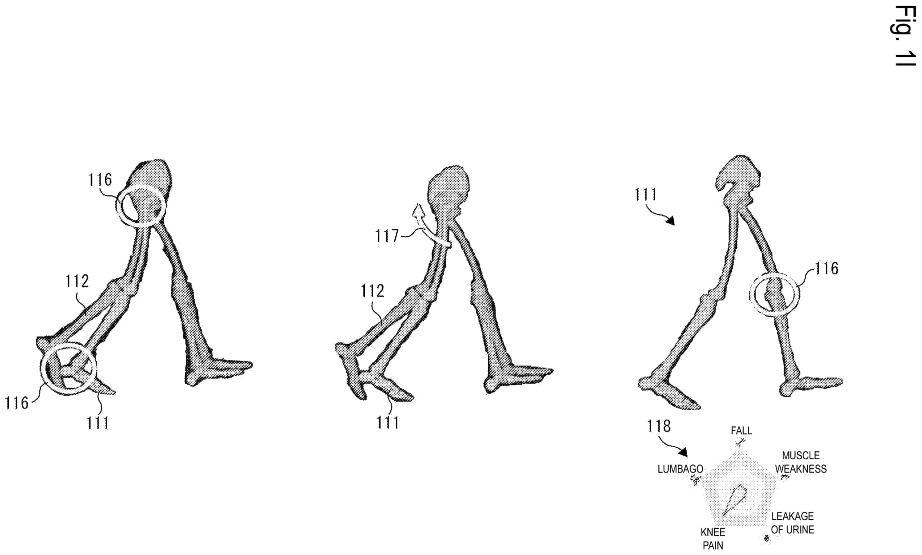

Further, as shown in Fig. 1I, the walking motion visualization apparatus 100 may display the skeleton model image 111 of the subject 110 and a skeleton model image 112 to be aimed at by the subject 110 in parallel, and may further display a place to be noticed by the subject 110 with circles 116 or the like (the left diagram in Fig. 1I). By displaying the images in parallel, the subject 110 can compare his or her walking motion with the walking motion to be aimed at, and thus it is possible to address an improvement in walking while more clearly noticing the improvement.

The walking motion visualization apparatus 100 may display the skeleton model image 111 of the subject 110 and the skeleton model image 112 to be aimed at by the subject 110 in parallel, and may further display a place or the like to be noticed by the subject 110 with an arrow 117 or the like (the center diagram in Fig. 1I). In this manner, an arrow may be displayed to indicate the direction and magnitude of movement so that a position at which the movement of a joint or the like of the subject 110 is insufficient can be moved more greatly.

Furthermore, the walking motion visualization apparatus 100 displays, with the circle 116 or the arrow 117, a position where a risk such as an injury or a disorder may occur in the future in the skeleton model image 111 of the subject 110 when the walking motion is continued as is. The walking motion visualization apparatus 100 may display, for example, a radar chart 118 as a display of the probability of occurrence of a risk or the like below the skeleton model image of the subject 110 (the right diagram in Fig. 1I). In the radar chart 118, the probability of a D position of a joint having a risk of a disorder or the like is displayed in an enlarged manner. In this manner, by displaying the possibility of injury or the like that may occur when the current walking motion is continued, it is possible to motivate the subject 110 to improve the walking.

Note that a risk is estimated by estimating at least two of a stride, a walking speed, and a difference between the left and right sides from a joint angle, and using two parameters, preferably three parameters, of these parameters.

The walking motion visualization apparatus 100 can also move and display the skeleton model images 111 and 112 shown in Fig. 1G to Fig. 1I, and the like, as animation images. In this manner, by moving and displaying the skeleton model image 111 of the subject 110, the subject 110 can easily recognize the current state of his or her walking motion (walking posture). The walking motion visualization apparatus 100 can also display the skeleton model image 111 of the subject 110 in a moving manner and display the advice and the like described above in parallel. In this manner, by displaying a hint, advice, or the like for improving a walking motion to the subject 110, the subject 110 can easily recognize which joint or part should be paid attention to. When the walking motion visualization apparatus 100 is for displaying advice on how to move the joints together, the subject 110 can more easily improve his or her walking motion.

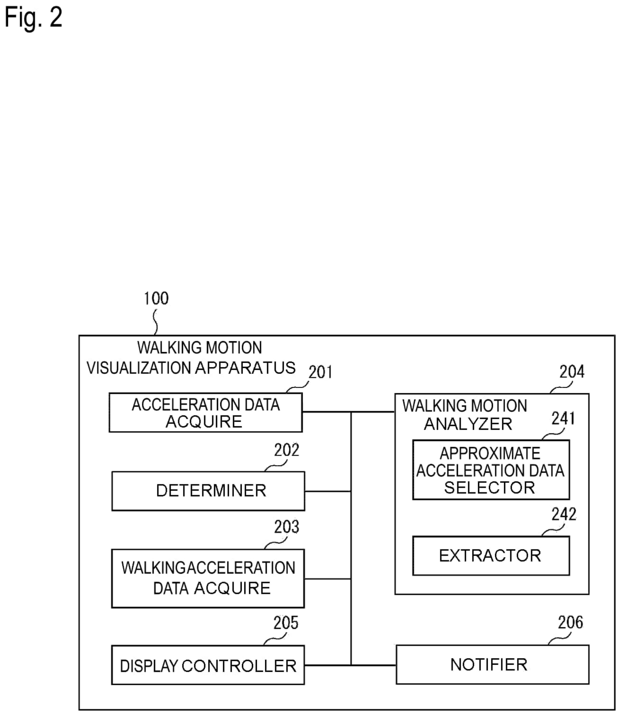

Next, the configuration of the walking motion visualization apparatus 100 will be described with reference to Fig. 2. The walking motion visualization apparatus 100 includes an acceleration data acquire 201, a determiner 202, a walking acceleration data acquire 203, a walking motion analyzer 204, a display controller 205, and a notifier 206. The walking motion analyzer 204 further includes an approximate acceleration data selector 241 and an extractor 242.

The acceleration data acquire 201 acquires stationary state acceleration data for determining the direction of gravity in a state where the subject 110 is stationary from an acceleration sensor disposed on a median line that is a center line between the left and right sides of the body of the subject 110. Similarly, the acceleration data acquire 201 acquires, from the acceleration sensor, determination acceleration data that is acceleration data when the subject 110 is walking, in order to determine whether the acceleration sensor is in a directly opposing state with respect to the median line or not.

First, the subject 110 holds the smartphone 120 including an acceleration sensor with both hands in accordance with a description displayed on a screen of the smartphone 120, positions the smartphone 120 in front of the abdomen of the subject's body, and maintains the smartphone 120 in a stationary state without moving the smartphone 120. In this state, when it is detected that there is no change in acceleration for a predetermined period of time, for example, two seconds, the acceleration data acquire 201 acquires the stationary state acceleration data.

Next, the walking motion visualization apparatus 100 prompts the subject 110 to walk several steps in a state where the subject 110 places the smartphone 120 in front of his or her abdomen. The walking motion visualization apparatus 100 prompts the subject 110 to walk several steps, for example, by outputting a sound for prompting the subject 110 to take an action from a speaker of the smartphone 120 or by vibrating the smartphone 120 using a vibration function. Then, the acceleration data acquire 201 acquires acceleration data while the subject 110 is walking several steps, as determination acceleration data for determining whether the acceleration sensor is in a directly opposing state with respect to the median line or not.

The determiner 202 determines whether the acceleration sensor is in a directly opposing state with respect to the median line or not on the basis of the acquired stationary state acceleration data and determination acceleration data. Specifically, the determiner 202 determines whether the acceleration sensor is in a directly opposing state or not on the basis of a comparison between a left-right axis component of the stationary state acceleration data and a left-right axis component of the determination acceleration data, between a front-back axis component of the stationary state acceleration data and a front-back axis component of the determination acceleration data, and between a vertical axis component of the stationary state acceleration data and a vertical axis component of the determination acceleration data.

The determiner 202 determines the direction of gravity by using the left-right axis component, the front-back axis component, and the vertical axis component of the stationary state acceleration data, that is, waveform data of a stationary state acceleration in each axis direction. Next, the determiner 202 determines a directly opposing state by comparing the left-right axis component, the front-back axis component, and the vertical axis component of the determination acceleration data, that is, waveform data of a determination acceleration in each axis direction with the determined direction of gravity or the like.

More specifically, the determiner 202 first determines the direction of gravity by using the left-right axis component, the front-back axis component, and the vertical axis component of the stationary state acceleration data, that is, the data of the stationary state acceleration in each axis direction. Next, a moving direction is determined using the determination acceleration data. Based on the moving direction and the vertical direction, the rotation axis angles of three axes are calculated by aligning a gravity vector and a vertical vector in a predetermined rotation order of the orthogonal axes, that is, the up-down, left-right, and front-back axes of the acceleration sensor. The directly opposing state is determined on the basis of the magnitude of the rotation angle.

As a method of determining the directly opposing state from each axis component (waveform data in each axis direction) of an acceleration obtained from the acceleration sensor, for example, a harmonic ratio (HR) can be used. HR is obtained by decomposing acceleration data (acceleration signal) of the left-right axis component, the front-back axis component, and the vertical axis component and calculating harmonics by discrete Fourier transform, and is obtained by discrete Fourier transform and calculating a ratio between an even frequency component and an odd frequency component. For example, when there is an axis component having an HR value of 0.9 or less among the axis components, it is determined that the axis component is away from the directly opposing state, and the subject 110 can be prompted to correct the position of the axis component. Then, when the value of HR of each axial component is 0.9 or more, the determiner 202 determines that the acceleration sensor (smartphone 120) is in a directly opposing state with respect to the median line of the body of the subject 110.

Note that it is preferable to prompt the subject 110 to correct the position, and to instruct the subject to hold the position when the position after correction is large when the value does not exceed 0.9, and to return the position to the original position, that is, the position before correction when the position before correction is large, thereby performing the subsequent measurement.

Further, the determiner 202 may estimate a central waveform from the waveform of the acceleration data obtained from the acceleration sensor by an algorithm or the like, for example, instead of using the value of HR. For example, an estimation formula for estimating the central waveform may be created by analyzing principal components of an R/L waveform. Alternatively, the determiner 202 may determine whether the acceleration sensor is in a directly opposing state or not by artificial intelligence (AI) using machine learning such as a support-vector machine (SVM) on the acceleration data. Note that a method of determining the directly opposing state is not limited to the method described here, and various methods can be used.

Note that, when an apparatus including a gyroscope, such as the smartphone 120, is used, the determiner 202 may determine a directly opposing state by further adding data of the gyroscope to the data of the acceleration sensor.

As a result, when the determiner 202 determines that the acceleration sensor is in a directly opposing state with respect to the median line, the walking acceleration data acquire 203 acquires walking acceleration data from the acceleration sensor while the subject 110 is walking. When it is determined that the acceleration sensor is in a directly opposing state, the walking motion visualization apparatus 100 may output, for example, a sound or the like prompting the subject 110 to start walking from, for example, the speaker of the smartphone 120. Note that, instead of outputting a sound, a message for prompting the subject to start walking may be output to a display or the like, or the subject may vibrate the smartphone using a vibration function. The subject 110 starts a walking motion in accordance with an output from the speaker or the vibration, and the walking acceleration data acquire 203 acquires acceleration data measured by the acceleration sensor thereafter as walking acceleration data of the subject 110 during walking.

The walking motion analyzer 204 analyzes the walking motion of the subject 110 on the basis of the acquired walking acceleration data. The walking motion of the subject 110 is analyzed by the walking motion analyzer 204 by selecting acceleration data approximate to the acquired walking acceleration data from a predetermined database. Here, the walking motion analyzer 204 further includes an approximate acceleration data selector 241 and an extractor 242.

First, the approximate acceleration data selector 241 compares a left-right axis component, a front-back axis component, and a vertical axis component of the acceleration data acquired by the walking acceleration data acquire 203 with a left-right axis component, a front-back axis component, and a vertical axis component of an acceleration stored in a predetermined database. Then, the approximate acceleration data selector 241 specifies acceleration data approximate to the acquired walking acceleration data. That is, the approximate acceleration data selector 241 specifies and selects a waveform of acceleration data approximate (similar) to the waveform of the walking acceleration data by matching the waveform (waveform feature) of the acquired walking acceleration data with the waveform (waveform feature) of the acceleration data stored in the predetermined database. Note that the approximate acceleration data selector 241 may specify and select a waveform of acceleration data that matches the waveform of the walking acceleration data.

The matching by the approximate acceleration data selector 241 can be performed using, for example, a Euclidean distance, a cosine distance, or the like between pieces of waveform data. Further, the approximate acceleration data selector 241 may perform the matching by performing calculation using dynamic time warping (DTW) as a distance measure in the tslearn of the machine-learning library, for example. That is, the matching can be performed by pattern-matching of time-series data in the tslearn (it is determined to be more approximate as the value approaches zero).

In addition, the matching by the approximate acceleration data selector 241 may be performed by arranging pieces of waveform data of accelerations and examining a correlation between the pieces of waveform data (the closer the value is to 1, the more approximate it is determined to be). In addition, for example, the approximate acceleration data selector 241 may perform matching using a joint angle of the subject 110 (AIST walking database 2019), or may perform matching by AI using machine learning such as SVM.

Next, the extractor 242 extracts joint angle data, which is a joint angle associated with the selected approximate acceleration data, from a predetermined database. Here, the predetermined database stores a relationship between the value of each component of the acceleration data and the joint angle. Then, the extractor 242 extracts, as an estimated value of a joint angle of the subject 110, joint angle data associated with the value of an acceleration (waveform data or the like) of each axis component stored in a predetermined database or the like.

Note that the predetermined database or the like to be used may include joint angle information of the upper body. In this case, unlike the above case, the joint angle of the upper body is estimated only from the data of the acceleration sensor on the median line.

The display controller 205 displays a walking motion model image 150 of the subject 110 on the basis of an analysis result obtained by the walking motion analyzer 204. The display controller 205 displays the walking motion model image 150 on, for example, a display of the smartphone 120 or the like, a display existing outside the walking motion visualization apparatus 100, or the like. The displayed walking motion model image 150 may be a still image or a moving image such as an animation.

The display controller 205 also displays a skeleton model image of the lower body of the subject 110 as the walking motion model image 150. Note that the display controller 205 may display, for example, a model image of the lower body of the subject 110 (an image to which not only the skeleton but also muscles, skin, and the like are added) in addition to the skeleton model image of the lower body of the subject 110.

Note that it is needless to say that, when the joint angle information of the upper body estimated using the data from the acceleration sensor or the database can be used, the upper body can be displayed.

Furthermore, the display controller 205 displays guidance information for guiding the subject 110 to a walking motion suitable for the subject, together with the walking motion model image 150 (the skeleton model image of the lower body). For example, the display controller 205 can also display advice for improving the degree of forward inclination and backward inclination, the left-right balance, and the walking motion of the subject 110, together with the skeleton model image. The display controller 205 may display the circles 113 and 116 indicating the positions of joints to be moved and the arrows 114 and 117 (the direction of an arrow and the thickness of a line) indicating the direction and amount to move a joint, thereby displaying information for guiding the way of moving the joint and the like. In addition, the display controller 205 may display, together with the walking motion model image 150, a number or the like indicating the order of moving joints, muscles, and the like so that a walking motion suitable for the subject 110 can be realized.

The notifier 206 issues a notification of the start of walking when the determiner 202 determines that the acceleration sensor is in a directly opposing state with respect to the median line and measurement is possible. A walking start instruction, notification of which is issued by the notifier 206, is, for example, vibration, a sound, light, or the like, but is not limited thereto.

In addition to vibrating the smartphone 120, the notifier 206 may output an approaching sound from, for example, the speaker of the smartphone 120 or a wireless earphone using electromagnetic waves or optical communication.

Note that the acceleration data acquire 201 and the determiner 202 may be taken out from the configuration of the walking motion visualization apparatus 100 to configure a directly opposing state determination apparatus for determining whether the acceleration sensor is in a directly opposing state with respect to the median line of the subject 110 or not.

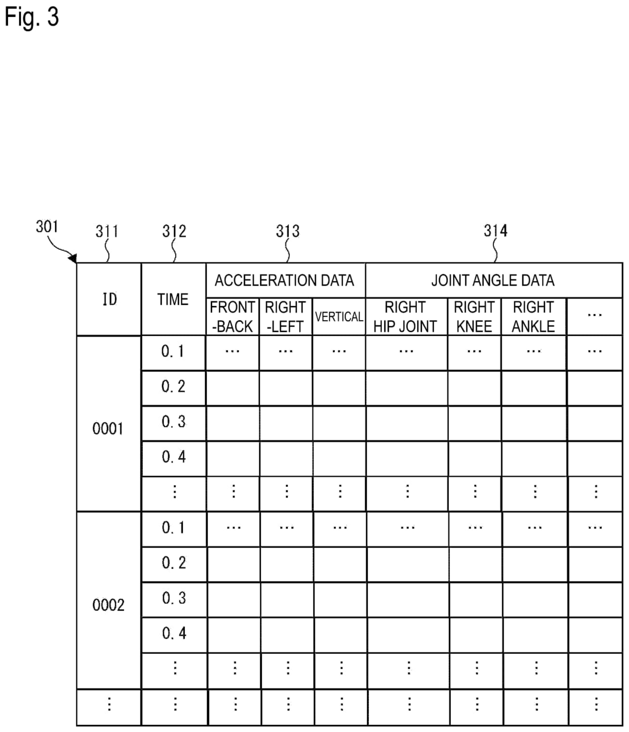

Next, an example of a joint angle table 301 included in the walking motion visualization apparatus 100 will be described with reference to Fig. 3. The joint angle table 301 stores a time 312, acceleration data 313, and joint angle data 314 in association with an identifier (ID) 311. The ID 311 is an identifier for identifying various types of data such as the time 312. The time 312 is a time at which the acceleration data 313 is measured. The acceleration data 313 is data measured by an acceleration sensor. The joint angle data 314 is data of the angle of each joint corresponding to the acceleration data measured by the acceleration sensor. These pieces of data may be data collected as so-called big data. Then, the walking motion visualization apparatus 100 extracts joint angle data approximate to the estimated joint angle of the subject 110 with reference to the joint angle table 301.

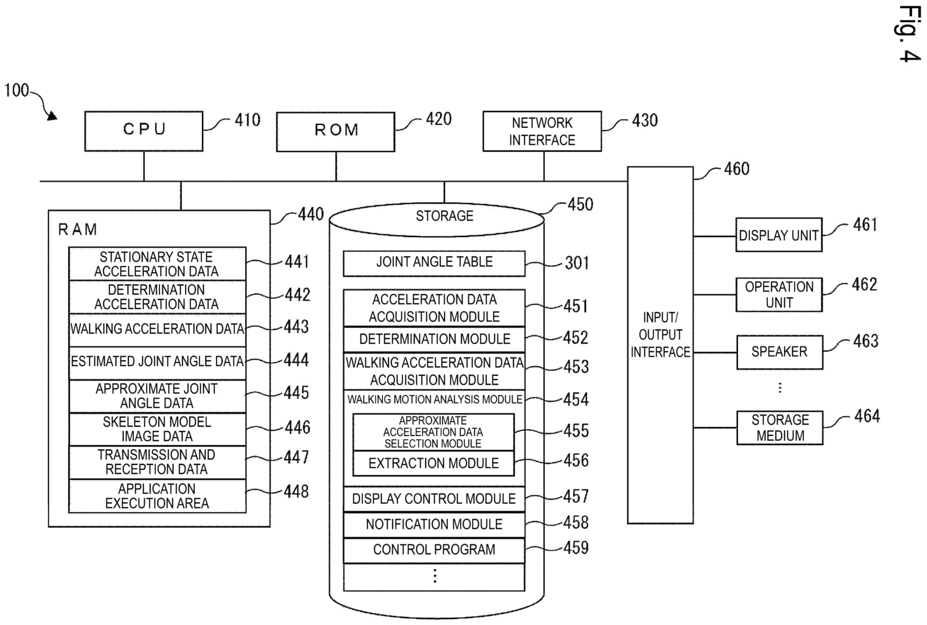

A hardware configuration of the walking motion visualization apparatus 100 will be described with reference to Fig. 4. A central processing unit (CPU) 410 is a processor for arithmetic control, and implements each functional configuration of the walking motion visualization apparatus 100 in Fig. 2 by executing a program. The CPU 410 may include a plurality of processors, and may execute different programs, modules, tasks, threads, and the like in parallel. A read only memory (ROM) 420 stores fixed data such as initial data and programs, and other programs. In addition, a network interface 430 communicates with other apparatuses via a network. Note that the CPU 410 is not limited to a single CPU, and may be a plurality of CPUs or may include a graphics processing unit (GPU) for image processing. In addition, it is desirable that the network interface 430 include a CPU independent of the CPU 410, and write or read out transmission and reception data in or from an area of a random access memory (RAM) 440. It is also desirable to provide a direct memory access controller (DMAC) (not shown) for transferring data between the RAM 440 and a storage 450. Further, the CPU 410 processes data by recognizing that the data has been received by or transferred to the RAM 440. The CPU 410 also prepares a processing result in the RAM 440, and subsequent transmission or transfer is handled by the network interface 430 or the DMAC.

The RAM 440 is a random access memory that is used as a temporary storage work area by the CPU 410. In the RAM 440, a storage area for storing data necessary for implementing the present embodiment is secured. Stationary state acceleration data 441 is data of acceleration measured by the acceleration sensor in a state where the subject 110 holds the acceleration sensor at a position directly opposing the median line of the front of the body of the subject. The determination acceleration data 442 is data of acceleration when the subject 110 walks in a state where the acceleration sensor held by the subject 110 is held at a position directly opposing the median line of the front of the body of the subject 110. Walking acceleration data 443 is data of the acceleration of the subject 110 during walking in a directly opposing state where the acceleration sensor is directly opposing the subject. Estimated joint angle data 444 is an estimated value of a joint angle of the lower body of the subject 110 during walking. Approximate joint angle data 445 is data of a joint angle approximate to the estimated joint angle of the subject 110, and is data extracted from a predetermined database or the like, for example. Skeleton model image data 446 is data representing the skeleton of the lower body of the subject 110 as the walking motion model image 150 of the subject 110, and is data for reproducing a walking motion of the subject 110 by a moving image or a still image.

Transmission and reception data 447 is data transmitted and received via the network interface 430. The RAM 440 also includes an application execution area 448 for executing various application modules.

The storage 450 stores a database, various parameters, or the following data or programs necessary for implementing the present embodiment. The storage 450 stores the joint angle table 301. The joint angle table 301 is a table for managing a relationship between the ID 311 and the acceleration data 313, the joint angle data 314, or the like shown in Fig. 3.

The storage 450 further stores an acceleration data acquisition module 451, a determination module 452, a walking acceleration data acquisition module 453, a walking motion analysis module 454, a display control module 457, and a notification module 458. The walking motion analysis module 454 further includes an approximate acceleration data selection module 455 and an extraction module 456. The acceleration data acquisition module 451 is a module that acquires stationary state acceleration data and determination acceleration data. The determination module 452 is a module that determines whether the acceleration sensor is in a directly opposing state with respect to the median line or not. The walking acceleration data acquisition module 453 is a module that acquires walking acceleration data of the subject 110 during walking when the acceleration sensor is in a directly opposing state. The walking motion analysis module 454 is a module that analyzes the walking motion of the subject 110 on the basis of the walking acceleration data. The approximate acceleration data selection module 455 is a module that compares the acquired walking acceleration data with a predetermined database (for example, the joint angle table 301) and selects approximate acceleration data approximate to the walking acceleration data from the predetermined database. The extraction module 456 is a module that extracts joint angle data, which is a joint angle associated with the selected approximate acceleration data, from a predetermined database. The display control module 457 is a module that displays the walking motion model image 150 of the subject 110 on the basis of an analysis result of the walking motion of the subject 110. The notification module 458 is a module that issues a notification of an alert in accordance with a deviation from the directly opposing state of the acceleration sensor with respect to the median line. These modules 451 to 458 are read out to the application executing area 448 of the RAM 440 by the CPU 410 and executed. A control program 459 is a program for controlling the entire walking motion visualization apparatus 100.

An input/output interface 460 interfaces input/output data with input/output equipment. A display unit 461 and an operation unit 462 are connected to the input/output interface 460. A storage medium 464 may be further connected to the input/output interface 460. Furthermore, a speaker 463 which is a sound output unit, a microphone (not shown) which is a sound input unit, or a GPS position determiner may be connected. Note that programs and data related to general-purpose functions and other realizable functions of the walking motion visualization apparatus 100 are not shown in the RAM 440 and the storage 450 shown in Fig. 4.

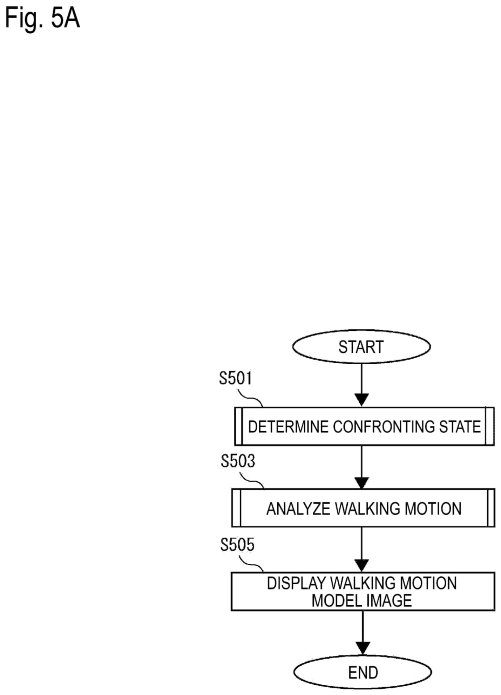

Next, a processing procedure of the walking motion visualization apparatus 100 will be described with reference to flowcharts shown in Fig. 5A to Fig. 5C. These flowcharts are executed by the CPU 410 in Fig. 4 using the RAM 440, and implement the functional components of the walking motion visualization apparatus 100 in Fig. 2.

First, a flow of the entire processing of the walking motion visualization apparatus 100 will be described with reference to Fig. 5A. In step S501, the walking motion visualization apparatus 100 determines whether the acceleration sensor held by the subject 110 in front of the median line of the body of the subject 110 is directly opposing the median line or not. In step S503, the walking motion visualization apparatus 100 analyzes a walking motion of the subject 110. In step S505, the walking motion visualization apparatus 100 displays the walking motion model image 150 on a display or the like on the basis of an analysis result of the walking motion.

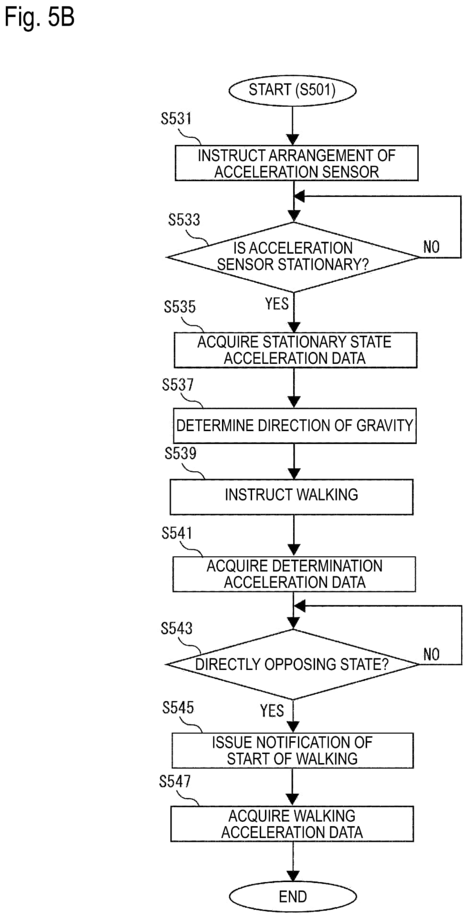

Next, a directly opposing state determination process of step S501 will be described in detail with reference to Fig. 5B. In step S531, the walking motion visualization apparatus 100 instructs the subject 110 to hold the acceleration sensor at a position directly opposing the median line of the body of the subject 110. In step S533, the walking motion visualization apparatus 100 determines whether the acceleration sensor is stationary or not. When it is determined that the acceleration sensor is not stationary (NO in step S533), the walking motion visualization apparatus 100 waits until the acceleration sensor is stationary. When it is determined that the acceleration sensor is stationary (YES in step S533), the walking motion visualization apparatus 100 proceeds to the next step.

In step S535, the acceleration data acquire 201 acquires stationary state acceleration data that is a measurement value in a state where the acceleration sensor is stationary. In step S537, the walking motion visualization apparatus 100 determines the direction of gravity from the acquired stationary state acceleration data. In step S539, the walking motion visualization apparatus 100 instructs the subject 110 to start walking. The walking motion visualization apparatus 100 instructs the subject 110 to walk several steps, for example. In step S541, the acceleration data acquire 201 acquires determination acceleration data, which is acceleration data for determining whether the acceleration sensor is in a directly opposing state or not. The determination acceleration data is acceleration data acquired while the subject 110 is walking several steps.

In step S543, the determiner 202 determines whether the acceleration sensor is in a directly opposing state with respect to the median line of the subject 110 or not. The determination regarding whether the acceleration sensor is in a directly opposing state or not is performed using, for example, the value of the above-mentioned HR (Harmonic Ratio). Then, when it is determined that the acceleration sensor is not in a directly opposing state (NO in step S543), the walking motion visualization apparatus 100 repeats the determination until the acceleration sensor is in an ecological state.

When it is determined that the acceleration sensor is in a directly opposing state (YES in step S543), the walking motion visualization apparatus 100 proceeds to step S545. In step S545, the walking motion visualization apparatus 100 notifies the subject 110 of the start of walking. In step S547, the walking acceleration data acquire 203 acquires walking acceleration data of the subject 110 during walking.

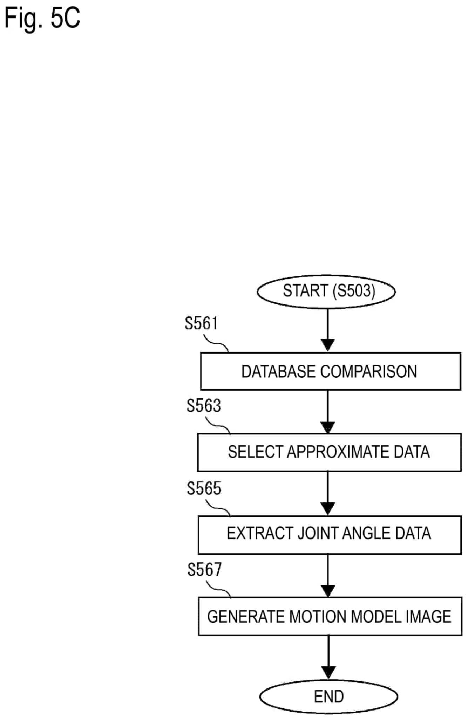

Next, details of the walking motion analysis process of step S503 will be described with reference to Fig. 5C. In step S561, the approximate acceleration data selector 241 compares the acquired walking acceleration data with a predetermined database. The comparison is performed, for example, for each of a left-right axis component, a front-back axis component, and a vertical axis component of the acquired walking acceleration data.

In step S563, the approximate acceleration data selector 241 selects approximate acceleration data that approximates the walking acceleration data as a result of the comparison of the respective axis components of the walking acceleration data. The selection of the approximate acceleration data is performed by, for example, a method of matching the acquired walking acceleration data with the acceleration data stored in the predetermined database, but the method is not limited to this method as long as the approximate acceleration data can be selected. Note that, when there is acceleration data that matches the walking acceleration data, the approximate acceleration data selector 241 may select the acceleration data as approximate acceleration data.

In step S565, the extractor 242 extracts joint angle data, which is a joint angle associated with the selected approximate acceleration data, from the predetermined database. In step S567, the walking motion visualization apparatus 100 generates the walking motion model image 150 of the subject 110 by using the extracted joint angle data. The generated walking motion model image 150 is, for example, a skeleton model image of the lower body of the subject 110. The walking motion visualization apparatus 100 also generates, as the generated skeleton model image, an animation image (moving image), a slide image (image obtained by combining a plurality of still images), or the like that reproduces a walking motion of the subject 110. Then, in step S505, the display controller 205 displays the generated skeleton model image on a display or the like.

According to the present embodiment, a walking motion of a subject is analyzed in a state where the acceleration sensor can output a correct measurement value, and thus it is possible to analyze the walking motion of the subject with high reliability, a small number of calculation steps, and a short calculation time. Further, since the walking motion of the subject is displayed to the subject using a skeleton model image, the subject can easily recognize points for improvement of the subject and the way of moving the body. Furthermore, when the acceleration sensor is in a directly opposing state with respect to the median line of the body of the subject, a walking motion is analyzed using an acceleration measured while the subject is walking, and thus it is possible to significantly reduce the amount of calculation, the number of calculation steps, and a calculation time in an analysis process for the walking motion. In this manner, since the amount of calculation and the like can be significantly reduced, the analysis result of the walking motion can be rapidly provided to the subject.

### Second Embodiment

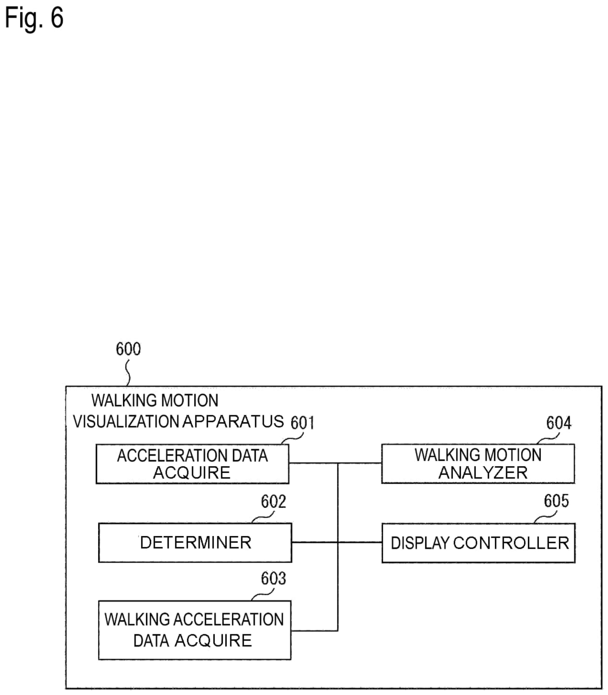

Next, a walking motion visualization apparatus according to a second embodiment of the present invention will be described with reference to Fig. 6. Fig. 6 is a block diagram showing the configuration of a walking motion visualization apparatus 600 according to the present embodiment.

The walking motion visualization apparatus 600 includes an acceleration data acquire 601, a determiner 602, a walking acceleration data acquire 603, a walking motion analyzer 604, and a display controller 605. The acceleration data acquire 601 acquires, from an acceleration sensor disposed on the median line which is the center line between the left and right sides of the body of the subject 110, stationary state acceleration data for determining the direction of gravity in a state where the subject 110 is stationary, and determination acceleration data which is acceleration data when the subject 110 is walking in order to determine whether the acceleration sensor is in a directly opposing state with respect to the median line or not. The determiner 602 determines whether the acceleration sensor is in a directly opposing state with respect to the median line or not on the basis of the acquired stationary state acceleration data and determination acceleration data. When the determiner determines that the acceleration sensor is in a directly opposing state with respect to the median line, the walking acceleration data acquire 603 acquires walking acceleration data of the subject 110 during walking from the acceleration sensor. The walking motion analyzer 604 analyzes the walking motion of the subject 110 on the basis of the acquired walking acceleration data. The display controller 605 displays a walking motion model image of the subject 110 on the basis of an analysis result of the walking motion analyzer.

According to the present embodiment, a walking motion of the subject is analyzed in a state where the acceleration sensor can output a correct measurement value, and thus it is possible to analyze the walking motion with high reliability.

While the invention has been particularly shown and described with reference to example embodiments thereof, the invention is not limited to these example embodiments. It will be understood by those of ordinary skill in the art that various changes in form and details may be made therein without departing from the spirit and scope of the present invention as defined by the claims. Further, a system or an apparatus in which different features included in the respective embodiments are combined in any way is also included in the scope of the present invention.

The present invention is applicable to a system including a plurality of devices or a single apparatus. The present invention is also applicable even when an information processing program for implementing the functions of example embodiments is supplied to the system or apparatus directly or from a remote site. Thus, the present invention also incorporates the program installed in a computer, a medium storing the program, a WWW (World Wide Web) server that causes a user to download the program. Especially, the present invention incorporates at least a non-transitory computer readable medium storing a program that causes a computer to execute processing steps included in the above-described example embodiments.

### Industrial Applicability

According to the present invention, a walking motion of a subject is analyzed in a state where an acceleration sensor can output a correct measurement value, and thus the walking motion can be analyzed with high reliability.

## Claims

1. A walking motion visualization apparatus comprising:
an acceleration data acquire for acquiring, from an acceleration sensor disposed on a median line that is a center line between left and right sides of a body of a subject,
stationary state acceleration data for determining a direction of gravity in a state where the subject is stationary and
determination acceleration data that is acceleration data when the subject is walking in order to determine whether the acceleration sensor is in a directly opposing state with respect to the median line;
a determiner for determining whether the acceleration sensor is in a directly opposing state with respect to the median line or not, based on the acquired stationary state acceleration data and determination acceleration data;
a walking acceleration data acquire for acquiring walking acceleration data from the acceleration sensor while the subject is walking when the determiner determines that the acceleration sensor is in a directly opposing state with respect to the median line;
a walking motion analyzer for analyzing a walking motion of the subject, based on the acquired walking acceleration data; and
a display controller for displaying a walking motion model image of the subject, based on an analysis result of the walking motion analyzer.

2. The walking motion visualization apparatus according to claim 1, wherein the determiner determines whether the acceleration sensor is in a directly opposing state or not, based on a comparison between a left-right axis component of the stationary state acceleration data and a left-right axis component of the determination acceleration data, between a front-back axis component of the stationary state acceleration data and a front-back axis component the determination acceleration data, and between a vertical axis component of the stationary state acceleration data and a vertical axis component of the determination acceleration data.

3. The walking motion visualization apparatus according to claim 1 or 2, wherein
the walking motion analyzer further includes:
an approximate acceleration data selector for selecting approximate acceleration data that is acceleration data approximate to the walking acceleration data by comparing the acquired walking acceleration data with a predetermined database, and
an extractor for extracting joint angle data, which is a joint angle associated with the selected approximate acceleration data, from the predetermined database, and
the display controller displays the walking motion model image using the extracted joint angle data.

4. The walking motion visualization apparatus according to any one of claims 1 to 3, further comprising a notifier for issuing a notification of start of walking when the determiner determines that the acceleration sensor is in a directly opposing state with respect to the median line.

5. The walking motion visualization apparatus according to any one of claims 1 to 4, wherein the display controller displays guidance information for guiding the subject to a walking motion suitable for the subject, together with the walking motion model image.

6. The walking motion visualization apparatus according to any one of claims 1 to 5, wherein the walking motion model image is a skeleton model image of a lower body of the subject.

7. A walking motion visualization method comprising:
an acceleration data acquisition step of acquiring, from an acceleration sensor disposed on a median line that is a center line between left and right sides of a body of a subject,
stationary state acceleration data for determining a direction of gravity in a state where the subject is stationary and
determination acceleration data that is acceleration data when the subject is walking in order to determine whether the acceleration sensor is in a directly opposing state with respect to the median line or not;
a determination step of determining whether the acceleration sensor is in a directly opposing state with respect to the median line or not, based on the acquired stationary state acceleration data and determination acceleration data;
a walking acceleration data acquisition step of acquiring walking acceleration data from the acceleration sensor while the subject is walking when it is determined in the determination step that the acceleration sensor is in a directly opposing state with respect to the median line;
a walking motion analysis step of analyzing a walking motion of the subject, based on the acquired walking acceleration data; and
a display control step of displaying a walking motion model image of the subject, based on an analysis result in the walking motion analysis step.

8. A directly opposing state determination apparatus comprising:
an acceleration data acquire for acquiring, from an acceleration sensor disposed on a median line that is a center line between left and right sides of a body of a subject,
stationary state acceleration data for determining a direction of gravity in a state where the subject is stationary and
determination acceleration data that is acceleration data when the subject is walking in order to determine whether the acceleration sensor is in a directly opposing state with respect to the median line; and
a determiner for determining whether the acceleration sensor is in a directly opposing state with respect to the median line or not, based on the acquired stationary state acceleration data and determination acceleration data.

9. The directly opposing state determination apparatus according to claim 8, wherein the determiner determines whether the acceleration sensor is in a directly opposing state or not, based on a comparison between a left-right axis component of the stationary state acceleration data and a left-right axis component of the determination acceleration data, between a front-back axis component of the stationary state acceleration data and a front-back axis component of the determination acceleration data, and between a vertical axis component of the stationary state acceleration data and a vertical axis component of the determination acceleration data.

10. A directly opposing state determination method comprising:
an acceleration data acquisition step of acquiring, from an acceleration sensor disposed on a median line that is a center line between left and right sides of a body of a subject,
stationary state acceleration data for determining a direction of gravity in a state where the subject is stationary and
determination acceleration data that is acceleration data when the subject is walking in order to determine whether the acceleration sensor is in a directly opposing state with respect to the median line or not; and
a determination step of determining whether the acceleration sensor is in a directly opposing state with respect to the median line or not, based on the acquired stationary state acceleration data and determination acceleration data.
